# EUROPEAN PATENT APPLICATION

(11) **EP 3 295 860 A1**
(43) Date of publication of application: **21.03.2018**
(21) Application number: 16818040.4
(22) Date of filing: 30.06.2016
(51) Int. Cl.: A61B 1/00

(54) **INSERTION-APPARATUS TIP COVER AND INSERTION APPARATUS**

(30) Priority: 30.06.2015 JP 2015131680
(71) Applicant: Olympus Corporation, Hachioji-shi Tokyo 192-8507 (JP)
(72) Inventor: YAMAYA, Koji, Tokyo 192-8507 (JP)
(74) Representative: Schicker, Silvia
(86) International application number: PCT/JP2016/069485
(87) International publication number: WO 2017/002932

(57) **Abstract**

A distal cover being attached to a distal configuration portion provided on a distal side of an insertion section to be inserted into a body, a ring-shaped first cover which is made of an insulating material and which has a lock portion that is removably locked to the distal configuration portion; and a ring-shaped second cover which is made of an insulating material that is more flexible than the first cover and which is provided more on a proximal side than the first cover.

## Description

### FIELD

The present invention relates to a distal cover of an insertion instrument which is attached to a distal configuration portion provided at the distal end of an insertion section to be inserted into a body, and it also relates to an insertion instrument.

### BACKGROUND

In general, an insertion instrument such as an insertion section of an endoscope has an opening in a distal configuration portion so that a treatment instrument having various functions, for example, a high-frequency treatment instrument which performs a resection treatment or the like on a living tissue by use of a high-frequency current extends from the opening through a channel.

Because the distal configuration portion normally comprises a combination of metallic members, a high-frequency electric current may be propagated to the metallic members or the metallic members may be used as parts of an electric current return path when the inserted high-frequency treatment instrument is in contact with or vicinity to the metallic members. Thus, when the high-frequency treatment instrument is introduced into a living body, it is necessary to take shielding measures such that the high-frequency electric current is not applied to living tissues other than a treatment target. As such measures, a distal cover which covers the exposed metallic members is attached to the distal configuration portion of the insertion section. For example, PATENT LITERATURE 1: Jpn. Pat. Appln. KOKAI Publication No. 09-075295 suggests a distal cover which is formed into a cap shape by a resin material and which is provided with a lock portion to prevent falling. This distal cover has a function to plastically deform the lock portion to prevent reuse at the time of detachment.

### SUMMARY

Conventional distal covers are entirely made of a hard resin for secure lock to the distal configuration portion, and users therefore have trouble in attachment.

It is an object of the present invention to provide a distal cover of an insertion instrument and an insertion instrument having this distal cover which achieve both secure attachability to a distal configuration portion and safety.

According to an embodiment of the present invention, there is provided a distal cover of an insertion instrument, the distal cover being attached to a distal configuration portion provided on a distal side of an insertion section to be inserted into a body, the distal cover characterized by comprising: a ring-shaped first cover which is made of an insulating material and which has a lock portion that is removably locked to the distal configuration portion; and a ring-shaped second cover which is made of an insulating material that is more flexible than the first cover and which is provided more on a proximal side than the first cover.

Moreover, according to an embodiment of the present invention, there is provided an insertion instrument characterized by comprising: a distal configuration portion provided on a distal side of an insertion section to be inserted into a body; and a distal cover which is attached to the distal configuration portion, the distal cover comprising a ring-shaped first cover which is made of an insulating material and which has a lock portion that is removably locked to the distal configuration portion, and a ring-shaped second cover which is made of an insulating material that is more flexible than the first cover and which is provided more on a proximal side than the first cover.

### BRIEF DESCRIPTION OF DRAWINGS

FIG. 1 is a sectional view showing a sectional structure wherein a distal configuration portion to which a distal cover of an insertion instrument according to a first embodiment is attached is seen from above;
FIG. 2 is a diagram showing an external configuration wherein the distal cover is seen from above;
FIG. 3 is a diagram showing an external configuration wherein the distal cover is seen sidewise;
FIG. 4 is a diagram showing an external configuration wherein the separated distal cover is seen sidewise;
FIG. 5 is a diagram showing an external configuration wherein the fitted distal cover is seen sidewise;
FIG. 6 is a diagram showing an external configuration wherein the distal configuration portion from which the distal cover is removed is seen from above;
FIG. 7 is a diagram showing an external configuration wherein the distal configuration portion from which the distal cover is removed is seen sidewise;
FIG. 8 is a diagram illustrating the leakage of a high-frequency electric current in the distal configuration portion to which the distal cover of the insertion instrument is attached;
FIG. 9 is a sectional view showing a sectional structure wherein the distal configuration portion to which a distal cover of the insertion instrument according to a second embodiment is attached is seen from above;
FIG. 10 is a diagram showing an external configuration wherein the separated distal cover is seen from above;
FIG. 11 is a diagram showing an external configuration wherein the separated distal cover is seen sidewise;
FIG. 12 is a diagram showing an external configuration wherein the separated distal cover is seen sidewise;
FIG. 13 is a diagram showing an external configuration wherein a distal cover according to a modification of the second embodiment is seen sidewise;
FIG. 14 is a sectional view showing a sectional structure wherein the distal configuration portion to which a distal cover of the insertion instrument according to a third embodiment is attached is seen from above;
FIG. 15 is a sectional view showing a sectional structure wherein the distal configuration portion to which a distal cover of the insertion instrument according to a fourth embodiment is attached is seen from above;
FIG. 16 is a sectional view showing a sectional structure wherein the distal configuration portion to which a distal cover of the insertion instrument according to a fifth embodiment is attached is seen from above; and
FIG. 17 is a diagram showing a configuration example in which the distal configuration portion of the insertion instrument is applied to a distal configuration portion of an insertion section of an endoscope.

### DETAILED DESCRIPTION

Embodiments of the present invention are described in detail with reference to the drawings.

### [First Embodiment]

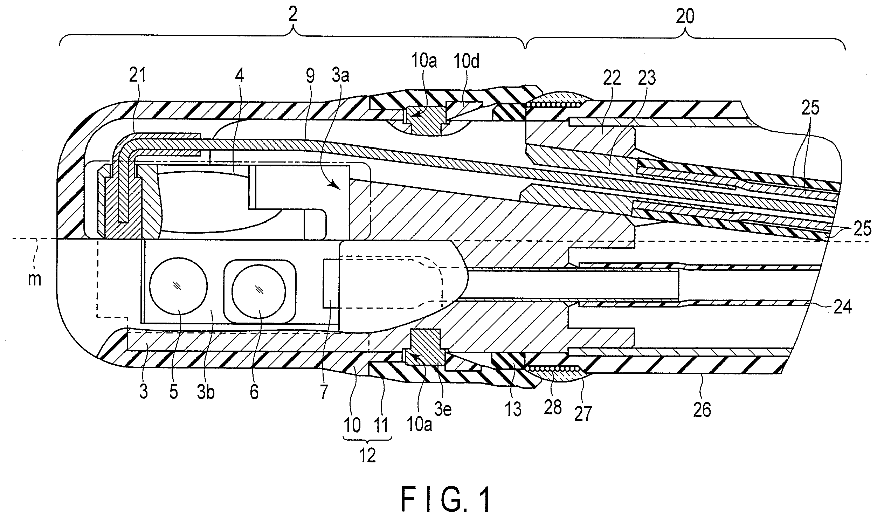

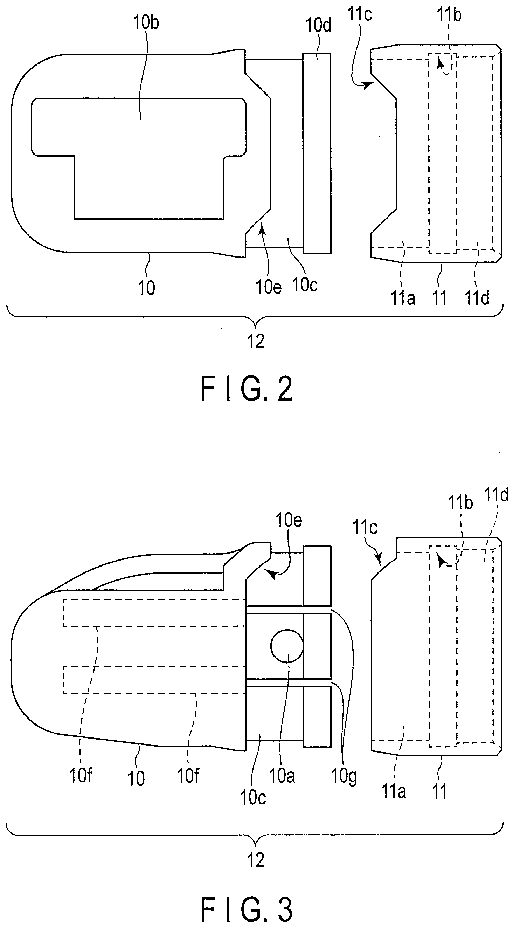

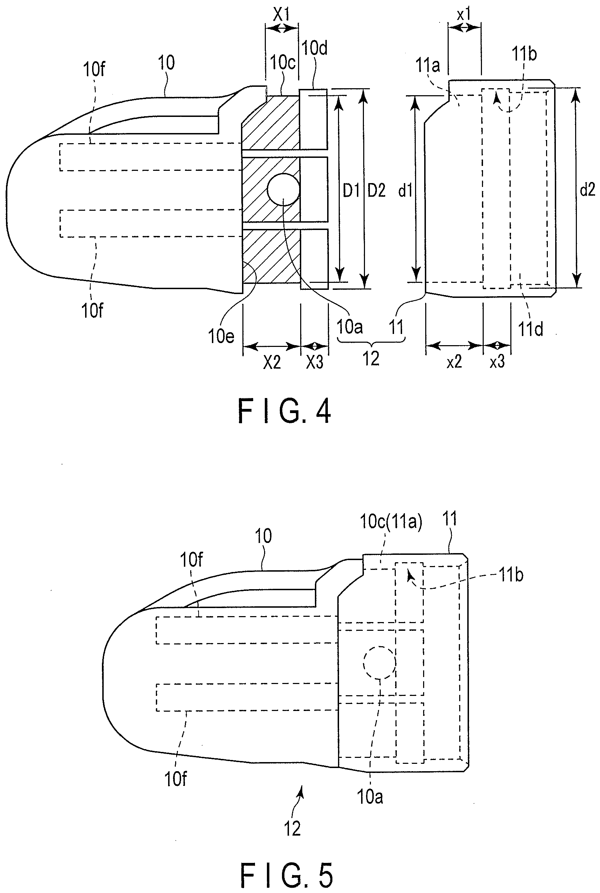

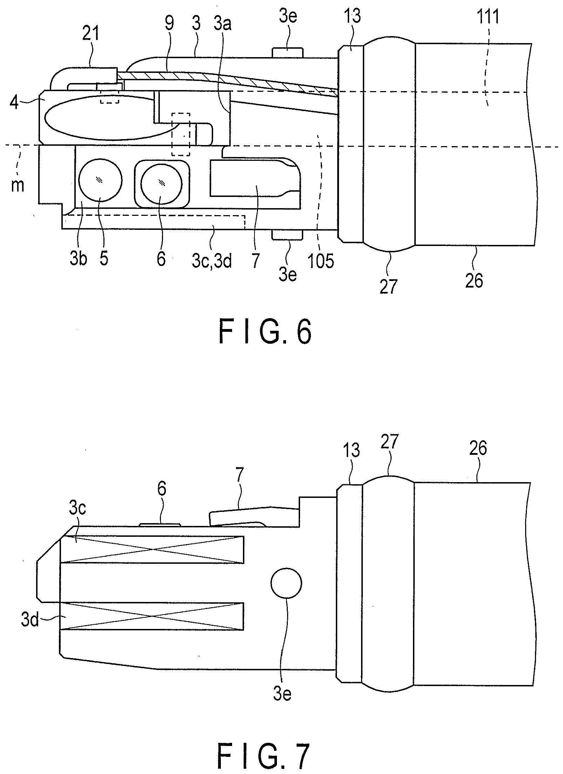

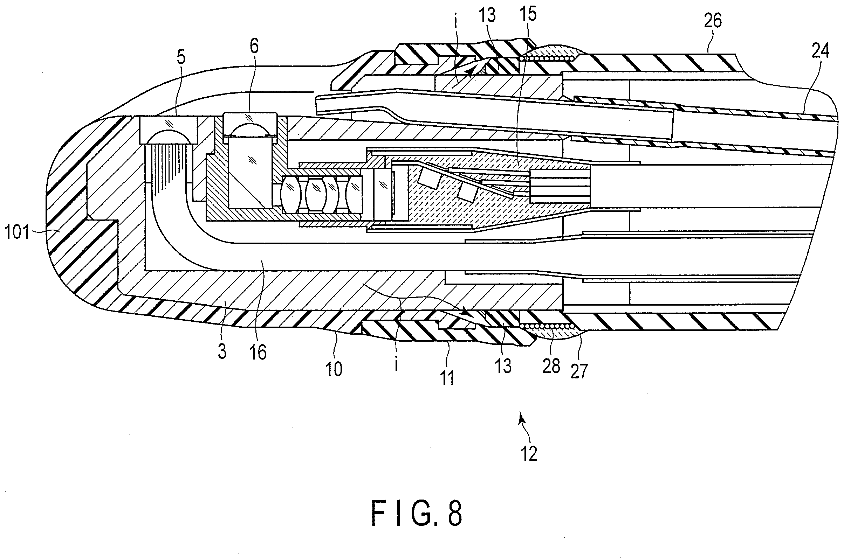

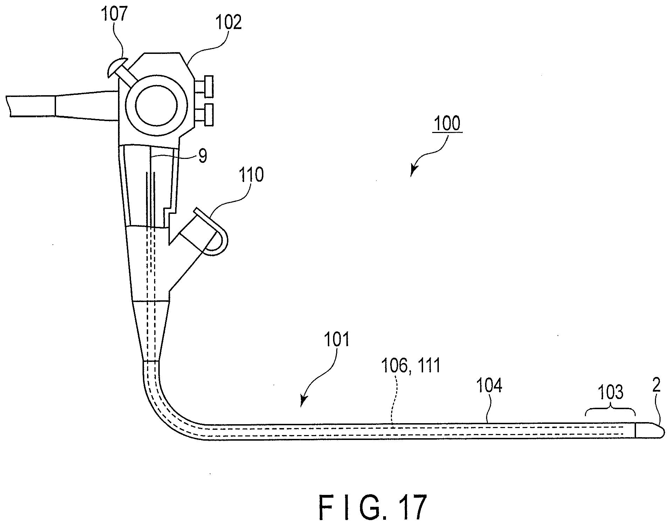

FIG. 1 is a sectional view showing a sectional structure wherein a distal configuration portion to which a distal cover of an insertion instrument according to the first embodiment is attached is seen from above. FIG. 2 is a diagram showing an external configuration wherein the distal cover is seen from above.FIG. 3 is a diagram showing an external configuration wherein the distal cover is seen sidewise. FIG. 4 is a diagram showing an external configuration to illustrate dimensional relations of the distal cover. FIG. 5 is a diagram showing an external configuration wherein the fitted distal cover is seen sidewise. FIG. 6 is a diagram showing an external configuration wherein the distal configuration portion from which the distal cover is removed is seen from above. FIG. 7 is a diagram showing an external configuration wherein the distal configuration portion from which the distal cover is removed is seen sidewise. FIG. 8 is a diagram illustrating the leakage of a high-frequency electric current. FIG. 17 is a diagram showing a configuration example in which the distal configuration portion of the insertion instrument according to the present embodiment is applied to a distal configuration portion of an insertion section of an endoscope.

The distal configuration portion of the insertion section of the endoscope shown in FIG. 17 is described by way of example as the distal configuration portion of the insertion instrument according to the present embodiment which is described below.

Described is a configuration example in which a member to change the movement direction of an inserted treatment instrument is mounted in this distal configuration portion. The member to change the movement direction includes, for example, a swing base which swings the treatment instrument, and a raising base which raises the treatment instrument. This swing base may be a swing mechanism which can be changed in a vertical direction, or in the vertical direction and a horizontal direction crossing the vertical direction. Although the treatment instrument is described as a high-frequency treatment instrument in each of the embodiments described below, the high-frequency treatment instrument is not limited thereto.

An endoscope 100 in FIG. 17 includes an insertion section 101 to be inserted into a body, and an operation section 102. On the proximal side of the insertion section 101, there is provided an insertion port (forceps opening) 110 of the treatment instrument which subjects a treatment target part to an electric treatment, for example, a treatment with a high-frequency electric current. A circular columnar distal configuration portion 2 is disposed on the distal side of the insertion section 101, and a swing base operation section 107 is provided in the operation section 102. Although not shown, this endoscope comprises a general system configuration including a light source device which supplies illumination light, a controller which includes a video processor that subjects an obtained video signal to image processing, a display section which displays observation images, a recorder which records video signals, and others.

In the insertion section 101, the distal configuration portion 2 is followed by a curving section 103 and a flexible tube 104 that are disposed on the proximal side. The insertion section 101 is internally equipped with a channel pipeline 111 through which the treatment instrument is inserted, an air/water supply pipe 106 to supply a washing solution and air, and a long tow wire (tow member) 9 for a swing operation (raising operation) of the swing base. The insertion section 101 is connected to the swing base operation section 107 via this tow wire 9. This tow wire 9 is connected to the swing base operation section 107 . A swing base 4 swings (turns) in response to the lever operation of the swing base operation section 107 so that the direction of the treatment instrument is changed.

The channel pipeline 111 is connected to a treatment instrument channel hole 105 (see FIG. 6) from the insertion port 110 on the proximal side through the inside of the insertion section 101, and the distal end of the treatment instrument channel hole 105 is open in the distal configuration portion 2 which will be described later. A channel opening 3a is open in the distal configuration portion 2. The high-frequency treatment instrument is put in from the insertion port 110, passes through the channel pipeline 111, protrudes (is exposed) at least in part from the channel opening 3a which communicates with the treatment instrument channel hole 105, and extends to the outside. Further, a recess is provided in the distal configuration portion 2, and the channel opening 3a may be open in the recess.

Now, the distal configuration portion 2 is described with reference to FIG. 1, FIG. 6, and FIG. 7.

It is preferable that this distal configuration portion 2 broadly comprises a base member 3 made of a metallic material such as stainless steel, the swing base 4 turnably provided in the base member 3, and a circular cylindrical distal cover 12. The base member 3 is fixed to an envelope member 26 of a distal side 20 (a part in which the curving section 103 is disposed) of the insertion section by the tying of a fixing thin wire 28. The envelope member 26 preferably comprises a water-tight tube made of an insulting resin or rubber. A fixing ring 27 coated with an insulating material is formed over the surface of the fixing thin wire 28. The insulating material includes, but is not limited to, resins or ceramics.

The base member 3 is divided into two parts along a longitudinal direction m of the insertion section. On one side, the channel opening 3a is disposed, and the swing base 4 is disposed in front of the channel opening 3a. On the other side, an observation surface 3b which is level to an outer peripheral surface is formed. The channel opening 3a communicates with the insertion port 110 on the proximal side through the channel pipeline 111.

Furthermore, an illumination window portion 5 through which illumination light guided by an optical fiber 16 (see FIG. 8) is emitted is disposed in the observation surface 3b. However, the material which guides light is not limited to the optical fiber, and an LED, for example, may be used.

An observation window portion 6 comprising an imaging section 15 (see FIG. 8) including an optical system, an imaging element, and an electric circuit is disposed adjacent to the illumination window portion 5. It is preferable that a nozzle portion 7 is provided on the proximal side of the longitudinal direction m in the vicinity of the observation window portion 6, and blows a washing solution such as physiological saline and a gas such as air supplied from an air/water supply pipe 24 (106) so that the illumination window portion 5 and the observation window portion 6 are suitably washed.

In the present embodiment, the observation window portion 6 permits the observation of a lateral side, namely, a peripheral direction of the distal configuration portion 2 that is a direction crossing the longitudinal direction m, and the illumination window portion 5 permits the application of the illumination light to the lateral side of the distal configuration portion 2 that is a direction in which observation is performed through the observation window portion 6. The crossing directions include, but are not limited in particular to, orthogonal directions.

That is, as an example of the present embodiment, this endoscope 100 is so-called a side-viewing observation endoscope or an oblique-viewing observation endoscope.

As shown in FIG. 6 and FIG. 7, groove-shaped rotation regulation portions 3c and 3d are formed to extend parallel to the longitudinal direction m in the side surface following at least the observation surface 3b in the base member 3. It is preferable that the rotation regulation portions 3c and 3d regulate rotation so that the distal cover 12 attached to the distal configuration portion 2 does not rotate in the peripheral direction. Further, a lock portion 3e to which a lock hole 10a of a later-described first cover 10 provided in the distal cover 12 fits is provided in an inner peripheral surface at a position more on the proximal side than the rotation regulation portions 3c and 3d. The lock portion 3e includes, for example, a circular columnar lock pin 3e, but the shape of the lock portion 3e is not limited to the circular columnar shape, and is not limited in particular as long as the lock portion 3e has a projecting and lockable shape.

It is preferable that the swing base 4 has a depressed abutment surface which abuts on the treatment instrument extending from the channel opening 3a and thus changes its movement direction, and a coupling portion 21 to which the tow wire 9 is coupled is provided on the outer surface of the swing base 4. The tow wire 9 is connected to the swing base operation section 107 through a wire guide member 25 which is fitted to a coupling member 23 fixed by a guide portion 22, and pushes and tows the coupling portion 21 by the lever operation so that the swing base 4 rises or falls accordingly. Moreover, an insulating ring 13 which is an insulating portion having insulating properties is formed over the entire periphery so that the insulating ring 13 is in close contact with the envelope member 26 and the end face of the fixing thin wire 28 by the base member 3. This insulating ring 13 is provided more on the proximal side of the flexible tube 104 than the lock pin 3e and the lock hole 10a that are the lock portions, that is, provided on an outer periphery of a joint part between the distal configuration portion 2 and the curving section 103 so that the insulating ring 13 has an outer peripheral surface adjacent to the distal configuration portion 2.

Now, the configuration of the distal cover 12 according to the present embodiment is described with reference to FIG. 2 and FIG. 3.

The distal cover 12 according to the present embodiment comprises the first cover 10 having two different actions and functions, and a second cover 11 provided more on the proximal side than the first cover 10. The first cover 10 and the second cover 11 may be adjacent to each other or may have some other member intervening therebetween.

The first cover 10 is made of an insulating material, and formed into a ring shape, for example, a bottomed cylindrical shape which is closed on the distal side. The bottomed cylindrical shape mentioned in the present invention has only to be a shape comprising a ring-shaped side surface and a bottom surface that covers one end of the side surface. The ring-shaped side surface of the first cover 10 is suitably set to a circular cylindrical shape, an elliptic cylindrical shape, or a polygonal cylindrical shape according to purposes. The bottom surface is not limited to a flat shape, and may have a semispherical shape or a tapered shape.

The second cover 11 has insulating properties. The second cover 11 is more flexible than the material of the first cover 10.The second cover 11 is ring-shaped. The ring-shaped side surface of the second cover 11 is suitably set to a circular cylindrical shape, an elliptic cylindrical shape, or a polygonal cylindrical shape according to purposes.

The material of the first cover 10 is preferably an elastic and plastic material having elasticity to deformation and plasticity. The first cover 10 is made of the elastic and plastic material and is thus deformed in a predetermined amount, but is broken beyond the predetermined amount.

Furthermore, the elastic and plastic material preferably includes, for example, a resin material such as plastics, and ceramics.

The resin material can be, but is not limited to, for example,methyl-methacrylate-acrylonitrile-butadiene-styrene , acrylonitrile-butadiene-styrene, polyvinyl chloride, polystyrene, polycarbonate, polypropylene, polysulfone, silicone rubber, Teflon (registered trademark) rubber, or fluororubber.

Moreover, the material of the second cover 11 is preferably elastic.

The second cover is preferably made of an elastic material, and is particularly preferably made of rubber.

The rubber material can be, but is not limited to, for example, silicone rubber, Teflon rubber, or fluororubber.

The first cover and the second cover may be made of different kinds of materials, or may be made of the same kind of material. When made of the same kind of material, the first cover and the second cover may have a difference of flexibility produced by, for example, the quantities of molecules, the quantities of cross-links, the quantities of additives, or the kinds of additives.

The second cover 11 part of the distal cover 12 may have a diameter which comes into close contact with the entire periphery of the target distal configuration portion 2, or may have a diameter with a predetermined space. The second cover 11 is more flexible than the first cover 10, and can therefore come into close contact with the distal configuration portion 2 by press force at the time of contact with a subject, and maintain electric safety, even if the space is formed between the second cover and the distal configuration portion 2.

The inside diameter of the second cover may be constant between the distal side and the proximal side, or may change. The minimum inside diameter of the distal cover 12 is, for example, 14 mm or less, and is preferably 13 mm or less.

The first cover 10 has a window 10b that is open to expose the swing base 4, the illumination window portion 5, and the observation window portion 6 provided in the distal configuration portion 2 to which the first cover 10 is attached. In the explanation below, the surface in which the window 10b is open is the upper surface. In addition, a direction from the distal side to the proximal side (open side) is the longitudinal direction, and a direction crossing (orthogonal to) the longitudinal direction is the peripheral direction.

It is preferable that a joint surface 10c which is one step lower than the outer peripheral surface is formed over the periphery on the proximal side of the first cover 10, and at its end on the open side, a fit portion 10d which has a step and is thus increased in thickness to have a lock function is provided. In addition, as shown in FIG. 1, the fit portion 10d may be formed into a tapered shape whose inner surface is wider on the open side so that the fit portion 10d is easily locked to the second cover 11 and easily fits to the distal configuration portion 2.

Furthermore, on both side surfaces of the joint surface 10c, the circular lock hole 10a or circular groove 10a which fits to the lock pin 3e of the distal configuration portion 2 is formed, and contributes to prevent falling. Moreover, more than one linear slit 10g extending in the longitudinal direction are formed on the both side surfaces of the joint surface 10c to elastically and outwardly expand the part including the lock hole 10a at the time of fitting to the distal configuration portion 2. Moreover, on the inner surface from the distal side of the first cover 10 to the joint surface 10c, rotation prevention protruding portions 10f comprising more than one bar-shaped protrusion (two protrusions in this example) which extend parallel to the longitudinal direction and which engage with the rotation regulation portions 3c and 3d are formed.

A proximal side abutment surface 11c of the second cover 11 is formed to conform to a distal side abutment surface 10e of the joint surface 10c of the first cover so that the outer peripheral surface of the second cover 11 smoothly links to the outer peripheral surface of the first cover 10.

Moreover, it is preferable that in the inner peripheral surface of the second cover 11 on the distal side, a joint surface 11a to come into close contact with the joint surface 10c when the second cover 11 is coupled to the first cover 10 (fitted to the first cover 10) is formed, and a fitting groove 11b which continues to the proximal side of the joint surface 11a and which fits to the fit portion 10d is formed.

Furthermore, the second cover 11 further protrudes toward the proximal side than the proximal end of the first cover 10, and the inner peripheral surface of the second cover 11 on the proximal side is formed as a joint surface 11d which fits to cover the outer peripheral surface of the insulating ring 13 provided adjacent to the distal configuration portion 2. The location of the insulating ring 13 is not limited, and may be included in the distal configuration portion 2.

It is preferable that the second cover 11 is flexible, and is formed to produce force on each part toward its center, for example, when the second cover 11 fits to the first cover 10 and covers the insulating ring 13. This force is, for example, elastic force, and can be produced by the reduction of the inside diameter of the second cover 11 compared to each of the outside diameters of the first cover 10 and the distal configuration portion 2. Here, one example of an attachment structure in the dimensions of the first cover 10 and the second cover 11 in the distal cover 12 is described with reference to FIG. 4.

Dimensional relations in the first cover 10 are D1≥d1, x1>X1, and x2>X2, wherein D1 is the diameter of the outer surface of the joint surface 10c of the first cover 10, d1 is the diameter of the inner surface of the joint surface 11a of the second cover 11 in a state where no force for diametrical increase is applied thereto, X1 is the minimum length of the upper surface of the joint surface 10c of the first cover 10 in the longitudinal direction, x1 is the minimum length of the upper surface of the joint surface 11a of the second cover 11, X2 is the maximum length of the joint surface 10c of the first cover 10,and x2 is the maximum length of the upper surface of the joint surface 11a of the second cover 11.

In this configuration, when the first cover 10 and the second cover 11 are fitted together, the joint surface 10c and the joint surface 11a are fitted together to come into close contact with each other without the use of an adhesive agent. The joint surface 10c and the joint surface 11a may come into close contact by a mechanical load, or may have an adhesive agent intervening therebetween as described below.

That is, the distal-side end of the second cover 11 overlaps the outer side of the proximal-side end of the first cover 10, and the proximal-side end of the first cover 10 is pressed by contractile force of the distal-side end of the second cover 11.

Another example of dimensional relations may be D1≥d1, x1>X1, x2>X2, D2>d2, and X3>x3, wherein X3 is the width of the fit portion 10d, and x3 is the width of the fitting groove 11b. Each of these dimensional relations is illustrative only, and the present invention is not limited exclusively to the relations described above.

Furthermore, in addition to the close contact by these dimensions, an adhesive agent (e.g. a silicon or epoxy adhesive agent) may be applied to the peripheries of the joint surface 10c and the distal side abutment surface 10e (and/or the joint surface 11a and the proximal side abutment surface 11c) shaded in FIG. 4 to bond the first cover 10 and the second cover 11 to each other. Moreover, the place to which the adhesive agent is applied is not limited in particular as long as the surfaces contact each other at this place. While the adhesive agent is not limited in particular, a silicon or epoxy adhesive agent, for example, can be used.

Moreover, it is possible to prevent the first cover 10 from coming off the lock pin 3e if the second cover 11 is configured to cover the lock pin and the lock hole 10a in a state where the lock pin 3e is in the lock hole 10a of the distal cover 12 when the distal cover 12 is attached to the distal configuration portion 2.

A procedure and advantageous effects of the attachment of the first cover 10 and the second cover 11 to the distal configuration portion 2 when the first cover 10 and the second cover 11 are separate bodies are described with reference to FIG. 1 to FIG. 5.

First, the second cover 11 is put in from the proximal side of the first cover 10 until the proximal side abutment surface 11c and the distal side abutment surface 10e conform to each other. The joint surface 10c and the joint surface 11a are brought into close contact, the fit portion 10d is fitted to the fitting groove 11b, and the first cover 10 and the second cover 11 are thereby coupled to each other to be integrated as the distal cover 12 so that the second cover 11 is disposed on the proximal side of the first cover 10.

Then the distal cover 12 is put into the distal configuration portion 2 from the distal side to the proximal side of the distal configuration portion 2 to cover the distal configuration portion 2, and put in until the joint surface 11d of the second cover 11 passes over the insulating ring 13 and is laid over (disposed at) the fixing ring 27 located more on the proximal side than the insulating ring 13. At this point, the first cover 10 enters in a state where the rotation prevention protruding portions 10f are fitted to the rotation regulation portions 3c and 3d, and the lock pin 3e of the distal configuration portion 2 is fitted into the lock hole 10a.

Accordingly, the second cover 11 is disposed to be coupled to the first cover 10 so that the second cover 11 is disposed more on the proximal side than the first cover 10 when the distal cover 12 is attached to the distal configuration portion 2, and the first cover 10 is locked to the distal configuration portion 2, whereby the distal cover 12 can be attached to the distal configuration portion 2. Further, the inner surface of the second cover 11 covers the outer surface of the insertion section 101, for example, the outer surface of the fixing ring 27 constituting the insertion section 101.

The inner surface of the second cover 11 may come into close contact with not only the fixing ring 27 but also the outer surface of a part which constitutes the insertion section 101 and which is greater in diameter than the periphery, for example, a connection portion between the distal configuration portion 2 and the curving section 103.

Although the first cover 10 and the second cover 11 are manufactured in separate manufacturing processes and then fitted and thereby integrated to form the distal cover 12 in the present embodiment, the present invention is not limited thereto. It is also possible to integrally manufacture the distal cover 12 by a continuous manufacturing process. For example, among injection molding manufacturing methods, heterogeneous material molding or so-called two-color molding can be used. According to this manufacturing method, first, a plastic material is injected into a primary side cavity to mold the first cover 10 to be a primary mold. The primary mold is subsequently put in a secondary side cavity, and then the rubber material is injected to integrally mold the second cover 11 which is coupled to the first cover 10. According to this manufacturing method, it is also possible to integrally manufacture the distal cover 12.
The first cover 10 and the second cover 11 may be made of the same material, and their flexibility may be adjusted by increasing the amount of ultraviolet application to the second cover part as compared to the amount of ultraviolet application to the first cover part, or by increasing the amount of heating.

Although the lock pin 3e of the distal configuration portion 2 is provided and then the lock hole 10a which fits to the lock pin 3e is formed in the first cover 10 in the configuration example described according to the present embodiment, the present invention is not necessarily limited to this configuration. For example, the lock pin 3e may be provided in the first cover 10, and then a lock hole which fits to the lock pin 3e may be formed on the distal configuration portion 2 side. That is, as long as the distal configuration portion 2 and the first cover 10 are locked by depressed and protruding configuration portions, it is also possible to provide a lock structure having a protruding member provided in the first cover 10 and a depressed member provided in the distal configuration portion 2.

Moreover, it is also possible to provide a lock structure in which a protrusion is provided on the outer surface side of the distal configuration portion 2, and a groove (lock portion) such as a lock hole which fits the protrusion is provided in the inner surface of the first cover 10.

Now, the flow of the high-frequency electric current of the distal configuration portion 2 is described with reference to FIG. 8.

The high-frequency treatment instrument passes through the inside of the insertion section 101 from the insertion port 110 shown in FIG. 17, and then extends from the channel opening 3a (see FIG. 6) of the distal configuration portion 2. When the high-frequency electric current is applied to the high-frequency treatment instrument to conduct a treatment, the high frequency is spatially propagated, and a high-frequency electric current i also flows in the distal configuration portion 2. At this point, the insulating ring 13 is covered with the second cover 11, so that the high-frequency electric current i does not leak out of the close contact portion and flow out of the distal cover 12. That is, the high-frequency electric current i does not leak out of the portion on the back side of the distal cover 12 that cannot be seen by a surgeon. Moreover, because the first cover 10 and the second cover 11 are in close contact with each other without any space even in their boundary, the high-frequency electric current does not leak out of the distal cover 12 from this part either.

In the present embodiment described above, the rotation of the distal cover 12 is prevented by the rotation prevention protruding portions 10f which fit to the rotation regulation portions 3c and 3d, and the fitting groove 11b and the lock hole 10a function to prevent coming-off as the distal cover 12 for the distal configuration portion 2 .Further, the fitting groove 11b and the lock hole 10a can be pressed by the fastening force of the second cover 11 so that the distal cover 12 does not easily come off the distal configuration portion 2.

The distal cover 12 includes the first cover 10, and the second cover 11 which is more flexible than the first cover 10, wherein the first cover 10 serves to lock to the distal configuration portion 2, and the second cover 11 serves to cover the insulating ring 13, so that both attachability and electric safety are achieved.

The distal cover 12 comprises the first cover 10 and the second cover 11 each having insulating properties, and can be attached to completely cover the part from the distal end of the base member 3 of the distal configuration portion 2 to the insulating ring 13 except the window 10b. It is therefore possible to prevent the leakage of the high-frequency electric current from the proximal side of the second cover 11, that is, the proximal side of the distal cover 12 as well as from the part covered with the distal cover 12.

That is, according to the distal cover in the present embodiment, it is possible to achieve both secure attachability and safety by attaching this distal cover to the distal configuration portion of the insertion instrument.

Furthermore, when the endoscope 100 is washed after used in a case, the distal cover 12 attached to the distal configuration portion 2 can be detached from the distal configuration portion 2 if the distal cover 12 (the first cover 10) is unlocked (decoupled) from the distal configuration portion 2 before the distal configuration portion 2, more particularly, the periphery of the swing base 4 is washed.

That is, the distal cover 12 (the first cover 10) can be removably locked to the distal configuration portion 2.

As described above, the first cover 10 is less flexible than the second cover 11, so that when the first cover 10 is attached to and detached from the distal configuration portion 2, the first cover 10 is not deformed or caught, and easy and secure attachability is obtained. Further, because the second cover 11 is made of the insulating material which is more flexible than the first cover 10, the second cover 11 can cover the proximal side of the distal cover 12 to prevent the possibility of the leakage of the high-frequency electric current.

### [Second Embodiment]

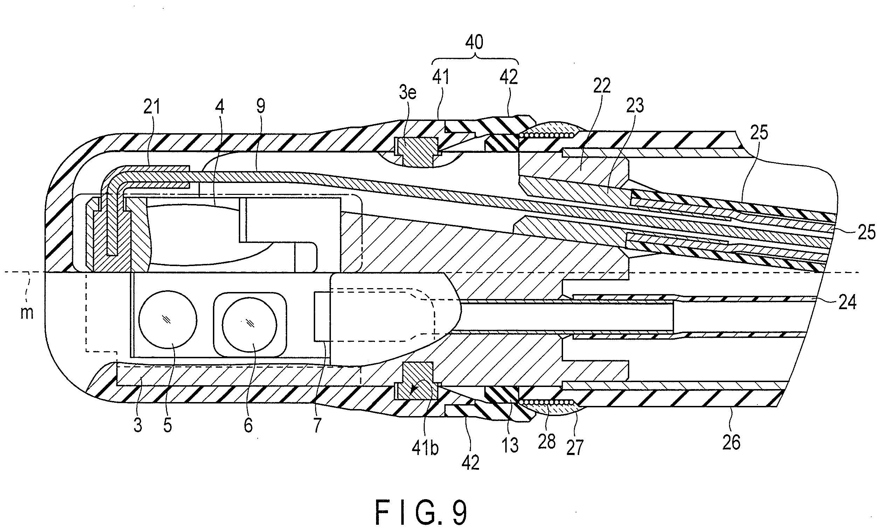

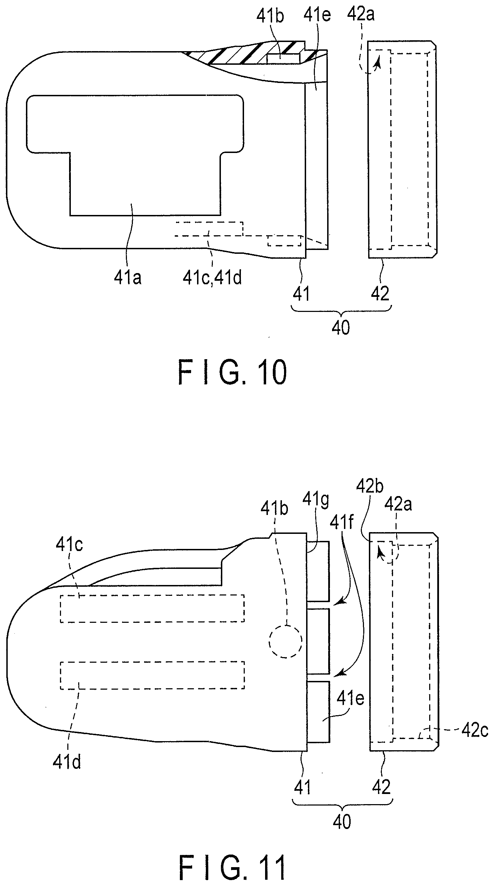

FIG. 9 is a sectional view showing a sectional structure wherein the distal configuration portion 2 to which a distal cover 40 is attached is seen from above. FIG. 10 is a diagram showing an external configuration wherein the distal cover 40 according to the second embodiment is seen from above. FIG. 11 is a diagram showing an external configuration wherein the distal cover 40 is seen sidewise. The distal configuration portion 2 according to the present embodiment is equivalent to that according to the first embodiment described above, and is indicated by the same reference number, and is not described.

The differences between the distal cover 40 according to the second embodiment and that according to the first embodiment are described.

The distal cover 40 originates in the same conception as the distal cover 12 described above, but the structure in the coupling (joint) portion between a first cover 41 and a second cover 42 is simplified, and the joint surface is changed to a smaller length. The configuration is similar in other respects .

A groove 41b which fits to the lock pin 3e of the distal configuration portion 2 is formed in the inner peripheral surface of the first cover 41, and contributes to prevent falling.

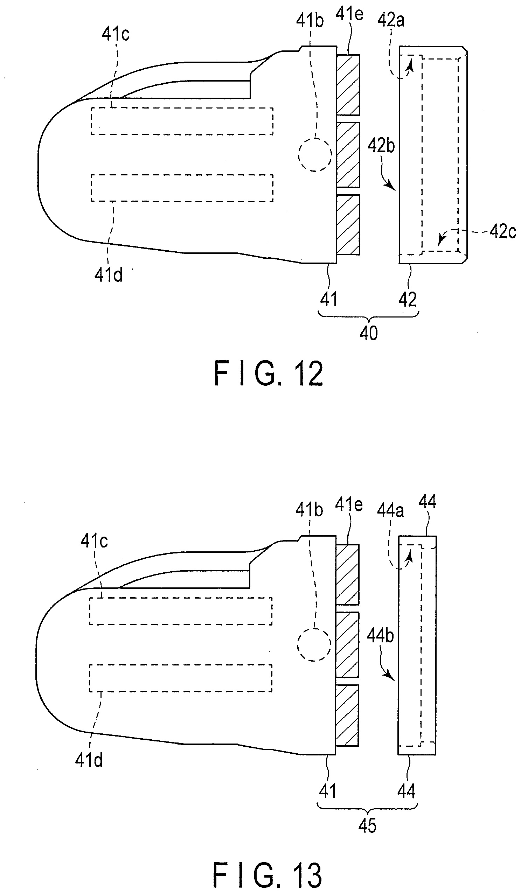

As shown in FIG. 11 and FIG. 12, a proximal side abutment surface 42b of the second cover 42 conforms to a distal side abutment surface 41g of the first cover 41 so that the outer peripheral surface of the second cover 42 smoothly links to the outer peripheral surface of the first cover 41. Further, the inner peripheral surface of the second cover 42 on the distal side is formed as a joint surface 42a to come into close contact with a joint surface 41e when coupled to the first cover 41 (fitted to the first cover 41) . The second cover 42 is disposed more on the proximal side than the proximal end of the first cover 41, and a joint surface 42c which is laid over the fixing ring 27 from the insulating ring 13 of the distal configuration portion 2 is provided on the proximal side following the aforementioned joint surface 42a.

The second cover 42 produces force on each part toward its center, for example, when the second cover 42 fits to the first cover 41 and covers the distal configuration portion 2. This force is, for example, elastic force, and can be produced by the reduction of the inside diameter of the second cover 42 compared to each of the outside diameters of the first cover 41 and the distal configuration portion 2.

A procedure and advantageous effects of the attachment of the distal cover 40 having such a configuration to the distal configuration portion 2 are similar to those in the first embodiment.

### [Modification of Second Embodiment]

FIG. 13 is a diagram showing an external configuration wherein a distal cover 45 according to a modification of the second embodiment is seen sidewise. The first cover 41 is equivalent to that according to the second embodiment described above, and is indicated by the same reference number, and is not described.

The distal cover 45 according to the present modification is substantially equivalent to the distal cover 40 described above, but a joint surface of a second cover 44 is changed to a smaller length than that of the second cover 42, and the structure of a joint part is simplified. The configuration is similar in other respects.

### [Third Embodiment]

A distal cover 50 according to the third embodiment is described.

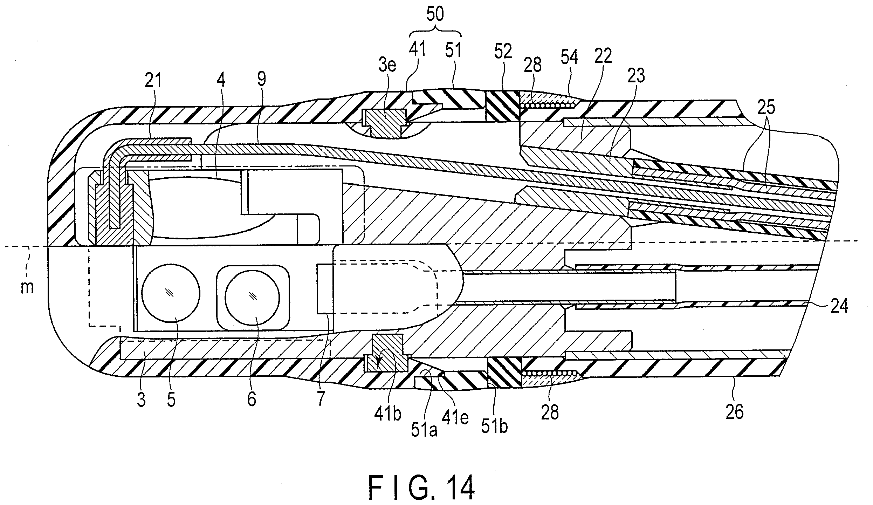

FIG. 14 is a sectional view showing a sectional structure wherein the distal configuration portion 2 to which the distal cover 50 according to the third embodiment is attached is seen from above. The distal configuration portion 2 according to the present embodiment is substantially equivalent except the positional relation between the insulating ring 13 according to the first embodiment described above and a second cover 51, and is indicated by the same reference number, and is not described.

In the present embodiment, the insulating ring 13 of the distal configuration portion 2 described above is increased in diameter to the height that smoothly links to the outer surface of the distal cover 50, and an insulating ring 52 which exposes the outer peripheral surface is formed. In the distal cover 50 according to the present embodiment, the first cover 41 is equivalent to the first cover 41 according to the second embodiment shown in FIG. 9, and is indicated by the same reference number, and is not described.

The structure of a joint part which is laid over a fixing ring portion 54 from the insulating ring 52 in the second cover 51 is further simplified, and a mechanism of abutment of the second cover 51 on the insulating ring 13 is changed to a joint surface 51b.

As shown in FIG. 14, the second cover 51 covers a step-shaped portion at the end of the first cover 41, and thereby fixes the first cover to the distal configuration portion 2. On the other hand, the step-shaped portion at the end of the first cover 41 presses the second cover 51 to the insulating ring 52 adjacent to the second cover 51.

The second cover 51 and the insulating ring 52 are adjacent and in close contact, thereby maintaining electric safety.

In this instance, the length of the second cover 51 in the longitudinal direction is formed to be greater than the distance between an end position (a position where the second cover 51 is laid) of the first cover 41 and the insulating ring 52. That is, the second cover 51 has flexibility to contract together with the first cover 41 between the first cover 41 and the insulating ring 52 and brings the first cover 41 and the insulating ring 52 into close contact with each other when attached to the distal configuration portion 2. In addition, the part in which a joint surface 51a and the joint surface 41e are in close contact may be bonded by the application of an adhesive agent.

According to the present embodiment, the leakage of the high-frequency electric current can be prevented by a simpler structure. Moreover, the area of exposure to the surface of the second cover 51 is smaller, and friction is reduced, leading to higher slidablity and higher insertability.

Furthermore, the second cover 11 comes into close contact to cover the insulating ring 13 and the fixing ring 27 from above in the arrangement and configuration according to the first embodiment described above, whereas in the configuration according to the present embodiment, the second cover 51 pushes from the side of the insulating ring 52 in the longitudinal direction, and then brings the second cover 51, the insulating ring 52, and the fixing ring portion 54 into close contact, so that there is no overlap, and the outside diameter can be smaller. Moreover, a lock hole 41b is inwardly pressed by fastening force of the second cover 51, and therefore, the distal cover 50 does not easily come off the distal configuration portion 2.

### [Fourth Embodiment]

A distal cover 60 according to the fourth embodiment is described.

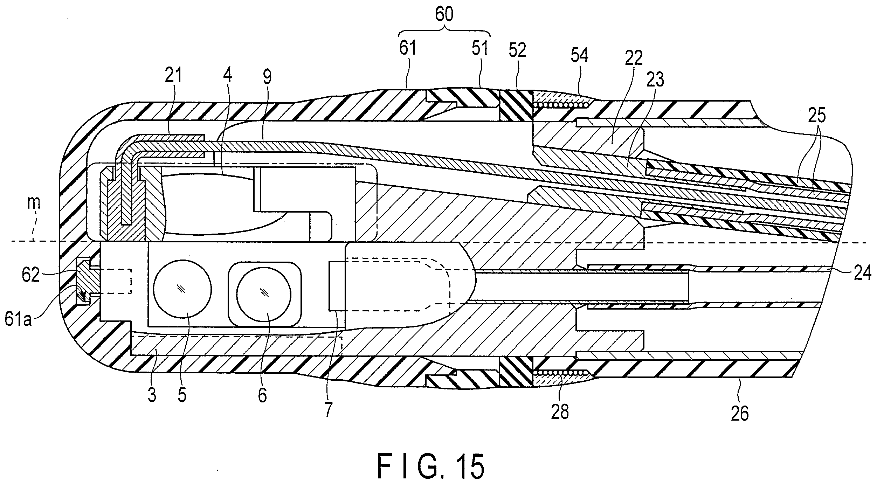

FIG. 15 is a sectional view showing a sectional structure wherein the distal configuration portion 2 to which the distal cover 60 according to the fourth embodiment is attached is seen from above. In the distal configuration portion 2 according to the present embodiment, as compared to the third embodiment described above, a joint configuration by a first cover 61 and a second cover 51 to prevent the leakage of the high-frequency electric current is equivalent, whereas the configuration to lock the first cover 61 and the distal configuration portion 2 is different.

A T-shaped lock pin 62 (lock portion) is provided at the distal end of the distal configuration portion 2. A lock groove 61a into which the T-shaped lock pin 62 is fitted is formed in the inner surface of the first cover 61 on the distal side. This T-shaped lock pin 62 has a chamfered or mushroom-shaped front corner of its distal portion, and thus easily enters the lock groove 61a and does not easily come off the lock groove 61a. When the first cover 61 is attached to the distal configuration portion 2, the T-shaped lock pin 62 pushes open and then enters an opening of the lock groove 61a, and is locked thereto. Although it is assumed that the top of the T-shaped lock pin 62 is disk-shaped, it is not limited as long as it has a shape that can fit to the lock groove 61a.

According to the present embodiment, a lock configuration by the first cover 61 and the distal configuration portion 2 is provided in the longitudinal direction, and the outside diameter of the first cover 61 can therefore be reduced. Further, the first cover 61 pushes the second cover 51 sidewise in the longitudinal direction, and then brings the second cover 51, the insulating ring 52, and the fixing ring portion 54 into close contact, so that the leakage of the high-frequency electric current to the outside can be prevented.

Furthermore, because of the configuration in which the second cover 51 comes into close contact from the side of the insulating ring 52 in the longitudinal direction, there is no overlap, and the outside diameter can be smaller. Moreover, a lock groove 61a does not easily expand because of the fastening force of the second cover 51, and therefore, the distal cover 60 does not easily come off the distal configuration portion 2.

### [Fifth Embodiment]

A distal cover 70 according to the fifth embodiment is described.

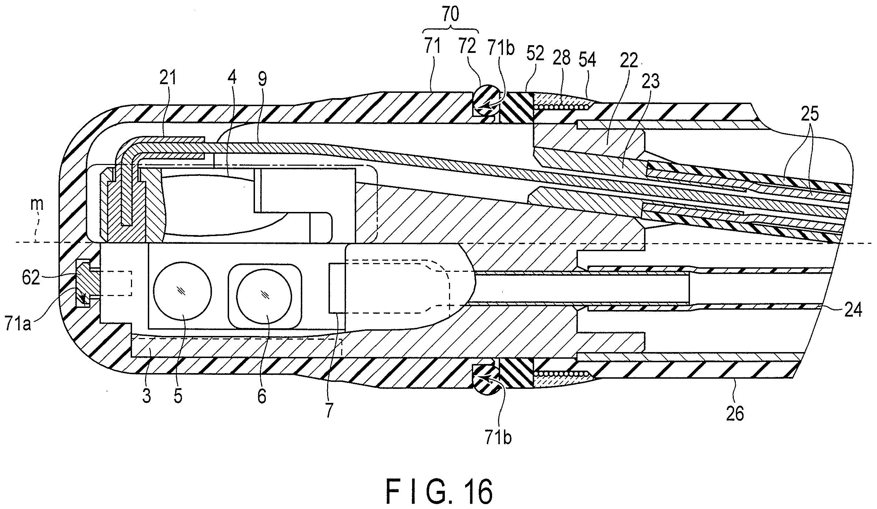

FIG. 16 is a sectional view showing a sectional structure wherein the distal configuration portion 2 to which the distal cover 70 according to the fifth embodiment is attached is seen from above. In the distal configuration portion 2 according to the present embodiment, as compared to the fourth embodiment described above, the configuration to lock a first cover 71 and the distal configuration portion 2 is equivalent, whereas a joint configuration by the first cover 71 and a second cover 72 to prevent the leakage of the high-frequency electric current is different.

As shown in FIG. 16, the T-shaped lock pin 62 (lock portion) is provided at the distal end of the distal configuration portion 2. A lock groove 71a into which the T-shaped lock pin 62 is fitted is formed in the inner surface of the first cover 71 on the distal side. Further, an L-shaped groove 71b is formed on the proximal side of the first cover 71. The second cover 72 comprises an O-ring made of insulating rubber. The second cover 72 inwardly presses the L-shaped groove 71b into close contact by contractile force (e.g. elastic force). Moreover, the T-shaped lock pin 62 holds the first cover 71 in the longitudinal direction, and thereby acts to flatten the O-ring of the second cover 72. Although the O-ring having a circular section is used in the present embodiment, the shape of the section is not limited thereto and may be rectangular or elliptic.

In the present embodiment described above, a lock configuration by the T-shaped lock pin 62 and the lock groove 71a is provided in the longitudinal direction, and the outside diameter of the first cover 71 can therefore be reduced.

Furthermore, the first cover 71 can be brought into close contact with the distal configuration portion 2 by the force (e.g. elastic force) of the second cover 72 comprising the O-ring. Additionally, the first cover 71 pushes the O-ring of the second cover 72 sidewise in the longitudinal direction, and then brings the first cover 71, the O-ring of the second cover 72, and the fixing ring portion 54 into close contact, so that the leakage of the high-frequency electric current to the outside can be prevented. Further, the O-ring is used as the second cover 72, the area of exposure to the surface is therefore smaller, and friction is reduced, leading to higher slidablity and higher insertability. Moreover, the lock groove 71a does not easily expand because of the fastening force of the second cover 72, and therefore, the distal cover 70 does not easily come off the distal configuration portion 2.

In addition, the distal cover according to each of the embodiments described above is preferably disposable and supposed to be used once, in which case it is preferable that the lock configuration part of the distal cover according to each of the embodiments and the distal configuration portion 2 is broken, and the distal cover (first cover) is thereby unlocked (decoupled) from the distal configuration portion 2 so that the distal cover can be detached from the distal configuration portion 2.

According to this configuration, the distal cover in each of the embodiments can be used only once in one case, leading to an improvement in the cleanliness of the endoscope 100. In addition, the endoscope 100, more particularly, the periphery of the swing base 4 after use in each case can be more easily washed, and safety can also be further increased.

Although not shown, the second cover 72 illustrated in the fifth embodiment may be combined with the lock portions illustrated in the first to third embodiments.

According to the present invention, it is possible to provide a distal cover of an insertion instrument, and an insertion instrument having such a distal cover, wherein the distal cover that blocks a high-frequency electric current and prevents its application is engaged with a distal configuration portion of the insertion instrument, which ensures secure attachment to the insertion instrument and also allows high safety to be maintained even in an operative method using the high-frequency electric current.

## Claims

1. A distal cover of an insertion instrument, the distal cover being attached to a distal configuration portion provided on a distal side of an insertion section to be inserted into a body, the distal cover **characterized by** comprising:
a ring-shaped first cover which is made of an insulating material and which has a lock portion that is removably locked to the distal configuration portion; and
a ring-shaped second cover which is made of an insulating material that is more flexible than the first cover and which is provided more on a proximal side than the first cover.

2. The distal cover of the insertion instrument according to claim 1, **characterized in that** the second cover has a predetermined length at which when the second cover is attached to the distal configuration portion, an inner surface of the second cover comes into close contact with an outer peripheral surface of the insertion section located more on the proximal side than an electric insulating portion provided adjacent to a proximal side of the distal configuration portion.

3. The distal cover of the insertion instrument according to claim 1, **characterized in that** the minimum inside diameter of the second cover is 14 mm or less.

4. The distal cover of the insertion instrument according to claim 1, **characterized in that** the first cover is made of an elastic and plastic material.

5. The distal cover of the insertion instrument according to claim 1, **characterized in that** the second cover is made of an elastic material.

6. The distal cover of the insertion instrument according to claim 1, **characterized in that** the lock portion is provided in an inner surface of the first cover, and
the lock portion fits to a groove or a protrusion provided in an outer surface of the distal configuration portion, and the first cover is thereby attached to the distal configuration portion.

7. The distal cover of the insertion instrument according to claim 1, **characterized in that** a distal-side end of the second cover overlaps an outer side of a proximal-side end of the first cover, and
the proximal-side end of the first cover is pressed by contractile force of the distal-side end of the second cover.

8. The distal cover of the insertion instrument according to claim 1, **characterized in that** the first cover and the second cover are attached to the distal configuration portion from a distal side of the distal configuration portion toward proximal side thereof.

9. An insertion instrument **characterized by** comprising:
a distal configuration portion provided on a distal side of an insertion section to be inserted into a body; and
a distal cover which is attached to the distal configuration portion, the distal cover comprising a ring-shaped first cover which is made of an insulating material and which has a lock portion that is removably locked to the distal configuration portion, and a ring-shaped second cover which is made of an insulating material that is more flexible than the first cover and which is provided more on a proximal side than the first cover.

10. The insertion instrument according to claim 9, **characterized in that** the insertion section has a channel through which a treatment instrument is passed to the body,
the channel has a channel hole which is open in the distal configuration portion,
the distal configuration portion includes a channel opening which communicates with the channel hole,
an electric insulating portion having an outer peripheral surface is provided adjacent to a proximal side of the distal configuration portion, and
the second cover covers the outer peripheral surface of the electric insulating portion or an outer peripheral surface of the insertion section located more on the proximal side than the electric insulating portion.

11. The insertion instrument according to claim 9, **characterized in that** the insertion section has a channel through which a treatment instrument is passed to the body,
the channel has a channel hole which is open in the distal configuration portion,
the distal configuration portion includes a channel opening which communicates with the channel hole, and
the distal configuration portion has a member which turns to change a movement direction of the treatment instrument protruding from a window provided in the distal configuration portion.

12. The insertion instrument according to claim 9, **characterized in that** an observation window is provided in a peripheral direction of the distal configuration portion, and
a window which exposes the observation window when the first cover is attached to the distal configuration portion is open in a peripheral direction of the first cover.
